# EUROPÄISCHE PATENTSCHRIFT

(11) **EP 1 124 783 B1**
(45) Veröffentlichungstag und Bekanntmachung des Hinweises auf die Patenterteilung: **21.05.2003**
(21) Anmeldenummer: 99959265.2
(22) Anmeldetag: 29.10.1999
(51) Int. Cl.: C07C 51/60, C07C 53/42, C07C 53/50

(54) **VERFAHREN ZUR HERSTELLUNG VON CARBONSÄURECHLORIDEN**
METHOD FOR PRODUCING CARBOXYLIC ACID CHLORIDES
PROCEDE DE PRODUCTION DE CHLORURES D'ACIDE CARBOXYLIQUE

(30) Priorität: 04.11.1998 DE 19850857
(43) Veröffentlichungstag der Anmeldung: 22.08.2001
(73) Patentinhaber: BASF AKTIENGESELLSCHAFT, 67056 Ludwigshafen (DE)
(72) Erfinder: STAMM, Armin, D-55128 Mainz (DE); HENKELMANN, Jochem, D-68165 Mannheim (DE)
(74) Vertreter: Isenbruck, Günter, Dr.
(86) Internationale Anmeldenummer: EP9908214
(87) Internationale Veröffentlichungsnummer: WO00026171

(56) Entgegenhaltungen:
- EP-A- 0 531 826
- US-A- 3 492 331
- US-A- 3 547 960

## Beschreibung

Die vorliegende Erfindung betrifft ein Verfahren zur Herstellung von Carbonsäurechloriden aus Carbonsäuren, Carbonsäureanhydriden, cyclischen Carbonsäureestern (Lactonen) oder Sulfonsäuren.

Carbonsäurechloride sind wichtige reaktive Zwischenprodukte bei der Herstellung von Fasern, Filmen sowie in der pharmazeutischen Chemie und Agrochemie. Daher sind eine Reihe von Herstellungsverfahren zur Synthese von Carbonsäurechloriden aus Carbonsäuren oder Carbonsäureanhydriden bekannt. In der industriellen Herstellung können drei Wege zur Herstellung von Carbonsäurechloriden unterschieden werden:
1. die Chlorierung von Carbonsäuren oder Carbonsäurenanhydriden mit Phosphortrichlorid,
2. die Chlorierung von Carbonsäuren oder Carbonsäurenanhydriden mit Thionylchlorid,
3. die Chlorierung von Carbonsäuren oder Carbonsäurenanhydriden mit Phosgen.

Die Chlorierung mit Phosgen ist gegenüber den anderen Verfahren vorteilhaft, da lediglich gasförmige Nebenprodukte anfallen, die leicht durch Austreiben mit beispielsweise Stickstoff entfernt werden können. Im Gegensatz zu Thionylchlorid wird nicht das toxische SO₂, sondern ungiftiges CO₂ gebildet. Des weiteren ist Phosgen ein preiswertes Chlorierungsmittel. Daher stellt die Chlorierung mit Phosgen eine sehr ökonomische Route zur Herstellung von Carbonsäurechloriden dar.

Da Phosgen allein bei geeigneten Reaktionstemperaturen und -drücken zu unreaktiv ist, ist die Verwendung eines Katalysators notwendig.

In der Literatur sind eine Reihe von Verfahren beschrieben, in denen N,N-disubstituierte Formamide oder deren Hydrochloride als Phosgenierungskatalysatoren eingesetzt werden. Diese setzen sich mit Phosgen zu den sogenannten Vilsmeier-Salzen um. Das Vilsmeier-Salz, das eigentlich reaktive Chlorierungsreagenz, reagiert mit einer Carbonsäure oder einem Carbonsäureanhydrid zum Säurechlorid. Dabei wird Formamid-Hydrochlorid zurückgebildet, das wiederum mit Phosgen zum Vilsmeier-Salz reagieren kann und weitere Katalysatorkreisläufe durchläuft. Die N,N-disubstituierten Formamid-Hydrochloride bzw. deren Vilsmeier-Salze sind jedoch thermisch nicht vollständig stabil, so daß es oberhalb von 80 bis 90°C zu Nebenreaktionen kommen kann.

In der EP-A 0 213 976 wird die Verwendung von Hexaalkylguanidiniumsalzen als Katalysatoren beschrieben. Diese müssen nur in geringen Mengen zur Reaktionsmischung zugegeben werden, um eine ausreichende Selektivität zu erreichen. Ein Nachteil dieser Verbindungsklasse ist jedoch deren aufwendige Herstellung.

In der US 3,547,960 ist die Verwendung von bestimmten Katalysatoren, die C-N-oder N-N-Doppelbindungen aufweisen, offenbart. Unter anderem werden cyclische Amidine als Katalysatoren offenbart. Bevorzugte Katalysatoren sind Imidazole oder Hydrochlorid-Salze davon und Triazole.

EP-A-0 531 826 offenbart die Herstellung von Carbonsäurechloriden aus Carbonsäuren unter Verwendung eines anorganischen Säurechlorides (O=AX₂, worin A: S oder C und X ein Halogen ist) unter Verwendung von ungesättigten Stickstoffverbindungen. Eine Guanidin-verbindung ist bevorzugt.

Aus Dokument US-A-3 492 331 sind Verbindungen der allgemeinen Formel III als Katalysator für die Phosgenierung von primären Aminen zu Isocyanaten bekannt.

Eine Aufgabe der vorliegenden Erfindung ist es, Katalysatoren für die Umsetzung von Carbonsäuren, Carbonsäureanhydriden, cyclischen Carbonsäureestern (Lactonen) oder Sulfonsäuren mit Phosgen zu Carbonsäurechloriden bereitzustellen,

Eine Aufgabe der vorliegenden Erfindung ist es, Katalysatoren für die Umsetzung von Carbonsäuren, Carbonsäureanhydriden, cyclischen Carbonsäureestern (Lactonen) oder Sulfonsäuren mit Phosgen zu Carbonsäurechloriden bereitzustellen, die gegenüber den bekannten Katalysatoren leichter herstellbar und für eine Vielzahl von Verbindungen anwendbar sind. Des weiteren sollen die Katalysatoren kürzere Reaktionszeiten als die bisher bekannten Katalysatoren bei sehr guten Umsätzen ermöglichen.

Diese Aufgabe wird gelöst durch ein Verfahren zur Herstellung von Säurechloriden durch Umsetzung von Carbonsäuren, Carbonsäureanhydriden, cyclischen Carbonsäureestern (Lactonen) oder Sulfonsäuren mit Phosgen in Gegenwart einer katalytischen Menge einer Verbindung aus der Gruppe bestehend aus N,N,N',N'tetrasubstituierten Amidinium-Halogeniden (I), N,N,N'-trisubstituierten Amidinium-Hydrohalogeniden (II) und N,N,N'-trisubstituierten Amidinen (III) der allgemeinen Formeln 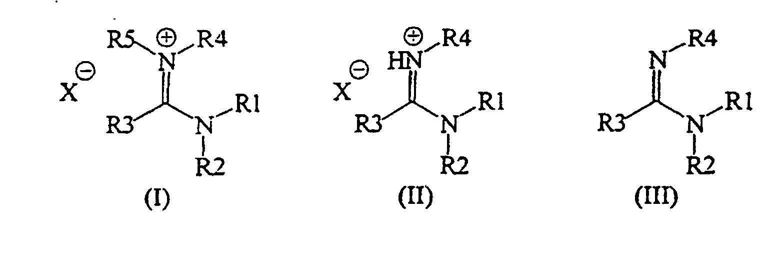 in denen R¹, R² und R⁴ lineare oder verzweigte Alkylketten der Länge C₁ bis C₂₀ oder ein cycloaliphatischer Rest mit einer Ringgröße von C₅ bis C₈ sind, wobei die Ringe durch Heteroatome unterbrochen sein können, oder R¹ und R² unsubstituierte oder substituierte aromatische Reste sind oder gemeinsam eine Kette aus vier oder fünf Methylengruppen aufspannen;
R³ ein Wasserstoffatom oder ein verzweigter oder unverzweigter Alkylrest oder Cycloalkylrest der Länge C₁ bis C₆; und

So können R¹, R² und R⁴ sowie R⁵ beispielsweise unabhängig voneinander Alkylreste wie Methyl-, Ethyl-, n-Propyl-, iso-Propyl-, n-Butyl-, iso-Butyl-, tert-Butyl oder lineare oder verzweigte Pentylreste sein. Die Reste R¹, R² und R⁴ können des weiteren Cycloalkylreste wie Cyclopentyl- und Cyclohexylreste sein und R¹ und R² können aromatische Reste, substituiert oder unsubstituiert, wie Phenyl- und Tolylreste sein.
R³ kann beispielsweise dieselben Alkylreste darstellen, die für R¹, R², R⁴ sowie R⁵ aufgeführt sind. Bevorzugt ist R³ jedoch Wasserstoff.
X⁻ ist bevorzugt Bromid oder Chlorid, ganz besonders bevorzugt Chlorid.

Besonders bevorzugt sind die Reste R¹, R², R⁴ und R⁵ Methyl- oder Ethylreste und R³ ist Wasserstoff. Besonders bevorzugt sind N,N,N'-Trimethylformamidin (R¹ = R² = R⁴ = Methyl, R³ = H), N,N,N'-Trimethylformamidin-hydrochlorid (R¹ = R² = R⁴ = Methyl, R³ = H), N,N,N',N'-Tetramethyl-formamidiniumchlorid (R¹ = R² = R⁴ = R⁵ = Methyl, R³ = H) und N,N-Diethyl-N',N'-dimethylformamidinium-hydrochlorid (R¹ = R² = Methyl, R⁴ = R⁵ = Ethyl, R³ = H).

Die in dem erfindungsgemäßen Verfahren eingesetzten Katalysatoren sind durch einfache Umsetzung auch im größerem Maßstab erhältlich. Sie ermöglichen eine Umsetzung in sehr guten Ausbeuten bei kurzen Reaktionszeiten.

Die N,N,N',N'-tetrasubstituierten Formamidinium-Salze (I) (R³ = H) sind durch eine einstufige Reaktion aus Formamiden mit N,N-disubstituierten Dialkylcarbaminsäurehalogeniden zugänglich. Eine Synthese der Verbindungen ist in Coll. Czech. Chem. Comm. 24 (1959), 760 bis 765 beschrieben.

N,N,N'-trisubstituierte Amidiniumhydrohalogenide (II) können durch Umsetzung von N-monosubstituierten Amiden mit N,N-Dialkylcarbaminsäurehalogeniden in einer einstufigen Synthese nach Kantlehner et al., Synthesis 1983 hergestellt werden. Die freien Amidine (III) können durch Neutralisation der so hergestellten Amidinhydrohalogenide mit einer anorganischen Base freigesetzt werden.

Da die Herstellung der freien Amidine (III) einen zusätzlichen Reaktionsschritt, nämlich die Neutralisation der Hydrohalogenide (II), erfordert, werden in dem erfindungsgemäßen Verfahren bevorzugt die Amidiniumhydrohalogenide der allgemeinen Formel II eingesetzt.

In einer weiteren bevorzugten Ausführungsform werden N,N,N',N'-tetrasubstituierte Amidiniumhalogenide (I), besonders bevorzugt Formamidiniumhalogenide, eingesetzt, die ebenfalls durch eine einstufige Umsetzung erhältlich sind, wie bereits beschrieben.

Die katalytische Wirkung der Amidiniumsalze I und II in dem erfindungsgemäßen Verfahren ist überraschend, da diese bisher als reaktionshemmend galten. So wird in US 3,547,960 als Nachteil von Verfahren, in denen Carboxamide als Katalysatoren eingesetzt werden, aufgeführt, daß bei dieser Reaktion durch die Zersetzung der Carboxamid-Katalysatoren teerige, katalytisch inaktive Produkte gebildet werden. Aus der Literatur ist jedoch bekannt, daß sich als Zersetzungsprodukt des Carboxamids DMF (Dimethylformamid) Formamidiniumhalogenide bilden.

Die Umsetzungen, in denen Amidine, Amidiniumhydrohalogenide bzw. Amidiniumhalogenide eingesetzt werden, gelingen um so besser, je mehr Substituenten die beiden Stickstoffatome aufweisen. Die Reaktion gelingt nicht vollständig, wenn keine Substituenten vorhanden sind (Vergleichsbeispiele 5 und 6). Bei zwei Substituenten an den Stickstoffatomen verläuft die Reaktion langsam, und es werden größere Mengen des Carbonsäureanhydrids gebildet (Vergleichsbeispiel 7).

Die in dem erfindungsgemäßen Verfahren einsetzbaren Carbonsäuren sind nicht eingeschränkt. Im allgemeinen werden aliphatische Carbonsäuren mit 2 bis 22 Kohlenstoffatomen oder Gemische von C₈- bis C₂₂- Carbonsäuren, deren Reste verzweigt oder linear, gesättigt oder ungesättigt und gegebenenfalls beispielsweise mit Halogen- oder Nitrogruppen substituiert sein können, eingesetzt. Des weiteren können aromatische und cycloaliphatische Carbonsäuren sowie aralkyl- oder alkylarylsubstituierte Carbonsäuren mit 7 bis 24 Kohlenstoffatomen eingesetzt werden. Geeignete Säuren können eine bis drei Carboxylgruppen enthalten. Geeignete aliphatische Säuren sind beispielsweise Pivalinsäure, 2-Ethylhexansäure, Stearinsäure, Buttersäure, Laurinsäure, Palmitinsäure, Essigsäure, Neopentansäure, Chloressigsäure, Dichloressigsäure, Adipinsäure, Sebacinsäure, Acrylsäure, Methacrylsäure usw.. Geeignete aromatische Säuren sind beispielsweise Benzoesäure, m-Nitrobenzoesäure, Isophthalsäure, Phenylessigsäure, p-Chlorbenzoesäure, trans-Zimtsäure, m-Toluylsäure usw.. Ein Beispiel für eine geeignete cycloaliphatische Säure ist Cyclohexancarbonsäure.

Für das erfindungsgemäße Verfahren geeignete Carbonsäureanhydride sind im allgemeinen Anhydride der allgemeinen Formel (IV) 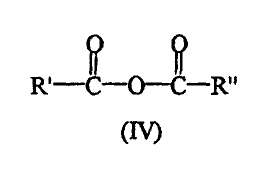 worin R' und R" jeweils einen organischen Rest, beispielsweise einen Kohlenwasserstoffrest, darstellen. Insbesondere sind Carbonsäureanhydride geeignet, die aliphatische Gruppen, die verzweigt oder linear, gesättigt oder ungesättigt und gegebenenfalls mit Halogen- oder Nitrogruppen substituiert sein können, oder cycloaliphatische oder aromatische Gruppen, sowie Aralkyl- oder Alkylarylgruppen tragen. So können R' oder R" Alkylen, Alkenylen, Cycloalkylen, Arylen oder eine divalente, gesättigte oder ungesättigte Gruppe sein. Bevorzugt beträgt die Zahl der Kohlenstoffatome in den aliphatischen Resten R' und R" 1 bis 24, besonders bevorzugt 1 bis 12 und in aromatischen, cycloaliphatischen, Aralkyl- oder Alkylarylresten 6 bis 24, bevorzugt 6 bis 12. So können als Anhydride beispielsweise Essigsäureanhydrid, Buttersäureanhydrid, Hexansäureanhydrid, Benzoesäureanhydrid, Trimellithsäureanhydrid, Octansäureanhydrid, Chloressigsäureanhydrid, Acrylsäureanhydrid, Phenylessigsäureanhydrid, Adipinsäureanhydrid, Sebacinsäureanhydrid, Nitrobenzoesäureanhydrid, Chlorbenzoesäureanhydrid, Toluylsäureanhydrid, Isophthalsäureanhydrid und Terephthalsäureanhydrid sein.

Für das erfindungsgemäße Verfahren geeignete cyclische Carbonsäureester (Lactone) sind im allgemeinen cyclische Ester der allgemeinen Formel V: 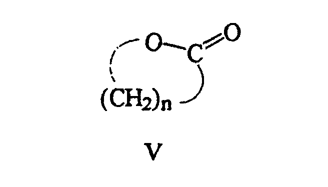 in denen n = 2 bis 9 ist.

Des weiteren sind auch ein- oder mehrfachsubstituierte cyclische Carbonsäureester geeignet, in denen ein oder beide Wasserstoffatome einer oder mehrerer CH₂-Gruppen durch Substituenten, insbesondere Alkylgruppen, ausgetauscht sind.

Besonders bevorzugt sind unsubstituierte cyclische Carbonsäureester, ganz besonders bevorzugt mit n = 3 bis 5. So sind beispielsweise γ-Butyrolacton, δ-Valerolacton und ε-Caprolacton geeignet.

Der Katalysator wird in dem erfindungsgemäßen Verfahren im allgemeinen in einer Menge von 0,1 bis 5, bevorzugt 0,5 bis 3, besonders bevorzugt 1 bis 2 mol-%, bezogen auf die Menge der eingesetzten Carbonsäure, des eingesetzten Carbonsäureanhydrids, des eingesetzten cyclischen Carbonsäureesters bzw. der eingesetzten Sulfonsäure, eingesetzt.

Das erfindungsgemäße Verfahren kann in Abwesenheit oder in Anwesenheit eines organischen, gegenüber Phosgen inerten Lösungsmittels, durchgeführt werden. Bevorzugte Lösungsmittel sind Kohlenwasserstoffe. Besonders bevorzugt sind einfach- oder mehrfach substituierte aromatische Kohlenwasserstoffe, ganz besonders bevorzugt sind Toluol, o-, m-, p-Xylol oder Chlorbenzol.

In einer besonders bevorzugten Variante, insbesondere bei Umsetzung von cyclischen Carbonsäureestern (Lactonen), wird als Lösungsmittel das gewünschte Carbonsäurechlorid eingesetzt.

Bevorzugt gegenüber der Durchführung des erfindungsgemäßen Verfahrens in Gegenwart eines Lösungsmittels ist jedoch die Abwesenheit von zusätzlichem Lösungsmittel. Dadurch wird eine Abtrennung des Lösungsmittels vom Reaktionsprodukt und somit ein Verfahrensschritt eingespart.

Das Verfahren kann in einer beliebigen, für die Umsetzung geeigneten Apparatur durchgeführt werden. So kann beispielsweise eine Phosgenierungsapparatur mit aufgesetztem Kohlensäurekühler eingesetzt werden. Die Durchführung des Verfahrens kann dabei diskontinuierlich oder kontinuierlich sein, wobei eine kontinuierliche Durchführung bevorzugt ist.

Die Reaktionstemperatur in dem erfindungsgemäßen Verfahren ist abhängig von der eingesetzten Carbonsäure bzw. dem eingesetzten Carbonsäureanhydrid. Im allgemeinen wird das erfindungsgemäße Verfahren bei Temperaturen von 40 bis 150°C, bevorzugt 60 bis 120°C, besonders bevorzugt 80 bis 100°C durchgeführt. Der Reaktionsdruck ist für die Umsetzung nicht kritisch. Im allgemeinen erfolgt die Umsetzung bei einem Druck von im allgemeinen 0,5 bis 5 bar, bevorzugt von 0,5 bis 2 bar, besonders bevorzugt 0,8 bis 1,2 bar, ganz besonders bevorzugt bei Normaldruck.

Die Reaktionszeiten liegen, in Abhängigkeit von der Menge und Art der eingesetzten Ausgangsverbindung, im Bereich von 1 bis 8 Stunden, bevorzugt im Bereich von 2 bis 4 Stunden. Die GC-Ausbeuten betragen im allgemeinen über 90 %, bevorzugt über 95 %, besonders bevorzugt über 98 %. Dabei sind GC-Ausbeuten gaschromatographisch bestimmte Ausbeuten. Die Umsätze sind üblicherweise nahezu vollständig.

Das Phosgen kann gasförmig oder in kondensierter Form zur Reaktionsmischung gegeben werden.

In einer bevorzugten Ausführungsform werden die Carbonsäure bzw. das Carbonsäureanhydrid und der Katalysator vorgelegt und auf die Reaktionstemperatur aufgeheizt. Anschließend wird solange Phosgen eingegast oder einkondensiert, bis die Umsetzung vollständig ist oder bis die eingegaste oder einkondensierte Phosgenmenge maximal der doppelten molaren Menge der eingesetzten Carbonsäure bzw. des eingesetzten Carbonsäureanhydrids entspricht. Für einen vollständigen Umsatz werden im allgemeinen insgesamt 1,0 bis 2,0 Äquivalente, bevorzugt 1,1 bis 1,5 Äquivalente, bezogen auf die molare Menge eingesetzter Carbonsäure bzw. eingesetztem Carbonsäureanhydrid, an Phosgen benötigt.

Die gebildeteten Säurechloride können mit dem Fachmann bekannten Methoden, wie Destillation, Ausfällung und Filtration und Umkristallisation usw., isoliert und aufgearbeitet werden. Bevorzugt werden die Säurechloride durch einfaches Abdekantieren von dem im Säurechlorid unlöslichen Katalysator abgetrennt. Eine solche Aufarbeitung ist einfach und kostengünstig und der eingesetzte Katalysator kann wiederverwendet werden.

Die nachfolgenden Beispiele erläutern die Erfindung zusätzlich.

### Beispiele

Die nachfolgenden Prozentangaben sind GC-Flächen-%.

### Beispiel 1:

In einer Phosgenierungs-Rührapparatur, umfassend einen 500 ml Vierhalskolben, sowie einen aufgesetzten Intensivkühler (Dimrothkühler gekühlt bei -10°C über Ministat und nachgeschalteter Kohlensäurekühler) werden 144 g (1 mol) 2-Ethylhexansäure und 1,28 g N-tert.-Butyl-N',N'-dimethylformamidin (Aldrich, 1 mol-%) vorgelegt und auf 75°C aufgeheizt. Innerhalb von 2 Stunden werden 110 g (1,1 mol) Phosgen bei einer Innentemperatur zwischen 75 und 85°C eingegast. Nach einer Nachreaktionszeit von 1 Stunde bei 80°C wird das überschüssige Phosgen bei 60°C mit Stickstoff ausgestrippt. Der Reaktionsaustrag enthält bei vollständigem Umsatz 98,9 GC-Flächen-% an 2-Ethylhexansäurechlorid und 0,2 GC-Flächen-% an 2-Ethylhexansäureanhydrid.

### Beispiel 2:

Beispiel 1 wurde mit 1 mol-% N,N,N'-Trimethylformamidin-Hydrochlorid als Katalysator wiederholt. Die Phosgenierung mit 105 g (1,05 mol-%) Phosgen erfolgt zwischen 80 und 90°C. Der Reaktionsaustrag enthält bei vollständigem Umsatz 98,5 % an 2-Ethylhexansäurechlorid und 0,6 % an 2-Ethylhexansäureanhydrid.

### Beispiel 3:

Beispiel 2 wurde mit 1,0 mol-% N,N,N',N'-Tetramethylformamidiniumchlorid als Katalysator wiederholt. Die Reaktion wurde zwischen 80 und 90°C durchgeführt. Der Reaktionsaustrag enthält bei vollständigem Umsatz 98,9 % an 2-Ethylhexansäurechlorid und 0,4 % an 2-Ethylhexansäureanhydrid.

### Beispiel 4:

Beispiel 3 wurde mit 1,0 mol-% N,N-Dimethyl-N',N'-diethyl-formamidiniumchlorid als Katalysator wiederholt. Der Reaktionsaustrag enthält bei vollständigem Umsatz 98,7 % an 2-Ethylhexansäurechlorid und 0,2 % an 2-Ethylhexansäureanhydrid.

### Beispiel 5 (Vergleichsbeispiel mit unsubsituiertem Amidin-hydrochlorid):

Beispiel 4 wurde mit 1,0 mol-% Benzamidin-hydrochlorid als Katalysator wiederholt. Der Reaktionsaustrag enthält 44,8 % unumgesetzte 2-Ethylhexansäure, 19,7 % 2-Ethylhexansäurechlorid und 35,3 % 2-Ethylhexansäureanhydrid.

### Beispiel 6 (Vergleichsbeispiel mit unsubstituiertem Amidin-hydrochlorid):

Beispiel 5 wurde mit 1,0 mol-% Acetamidin-hydrochlorid als Katalysator wiederholt. Der Reaktionsauftrag enthält 22,2 % unumgesetzte 2-Ethylhexansäure, 25,8 % 2-Ethylhexansäurechlorid und 51 % 2-Ethylhexansäureanhydrid.

### Beispiel 7 (Vergleichsbeispiel mit einem disubsituierten Amidin):

Beispiel 6 wurde mit 1,0 mol-% N,N'-Diphenylformamidin als Katalysator wiederholt. Der Reaktionsaustrag enthält 70,2 % an 2-Ethylhexansäurechlorid und 28,3 % an 2-Ethylhexansäureanhydrid.

### Beispiel 8: Phosgenierung eines cyclischen Esters (diskontinuierliche Fahrweise)

In einer Phosgenierungs-Rührapparatur aus einem 500 ml Vierhalskolben sowie einem aufgesetzten Intensivkühlsystem (Dimrothkühler, gekühlt bei -10°C mit nachgeschaltetem Kohlensäurekühler, -78°C) werden 129 g (1,5 mol) γ-Butyrolacton und 4,1 g (0,03 mol) Tetramethylformamidiniumchlorid (TMFCl) vorgelegt und auf 145°C erhitzt. Innerhalb von 4 h werden insgesamt 181 g (1,8 mol) Phosgen und ca. 40 g (10 g/h) HCl parallel so in die Lösung eingeleitet, daß die Reaktionstemperatur zwischen 145 und 150°C gehalten werden kann. Nach einer Nachreaktionszeit von 1 h bei 145°C wird das überschüssige Phosgen mit Stickstoff bei 80°C ausgestrippt. Der Reaktionsaustrag enthält nach gaschromatographischer Analyse 89,1 Flächen-% 4-Chlorbuttersäurechlorid und 5,0 Flächen-% γ-Butyrolacton.

### Beispiel 9: Phosgenierung eines cyclischen Esters (semi-kontinuierliche Fahrweise)

In einer Phosgenierungs-Rührapparatur aus einem 500 ml Vierhalskolben sowie einem aufgesetzten Intensivkühlsystem (Dimrothkühler, gekühlt bei -10°C, mit nachgeschaltetem Kohlensäurekühler, -78°C) werden 30 g (0,21 mol) Chlorbuttersäurechlond und 0,6 g (0,0042 mol) Tetramethylformamidiniumchlorid (TMFCl) vorgelegt und auf 145°C erhitzt. Bei 145 bis 150°C Innentemperatur werden über 6 h je 10 g/h Chlorwasserstoffgas und je 28 g/h (0,33 mol/h) γ-Butyrolacton (mmit 0,9 g (= 2 mol-%) TMFCI versetzt) sowie insgesamt 156 g (1,56 mol) Phosgen so eingeleitet, daß ein ständiger Phosgenrückfluß beibehalten wird. Nach dem Ende des Zulaufes werden weitere 38 g Phosgen bei 145 bis 150°C zum Nachreagieren eingeleitet. Nach Abkühlen auf 80°C wird mit Stickstoff überschüssiges Phosgen ausgestrippt. Der Reaktionsaustrag (284 g) enthält nach gaschromatographischer Analyse 87,8 Flächen-% 4-Chlorbuttersäurechlorid und 6,2 Flächen-% γ-Butyrolacton.

## Patentansprüche

1. Verfahren zur Herstellung von Säurechloriden durch Umsetzung von Carbonsäuren, Carbonsäureanhydriden, cyclischen Carbonsäureestern oder Sulfonsäuren mit Phosgen in Gegenwart einer katalytischen Menge einer Verbindung aus der Gruppe bestehend aus N,N,N',N'-tetrasubstituierten Amidinium-halogeniden (I), N,N,N'-trisubstituierten Amidiniumhydrohalogeniden (II) und N,N,N'-trisubstituierten Amidinen (III) der allgemeinen Formel 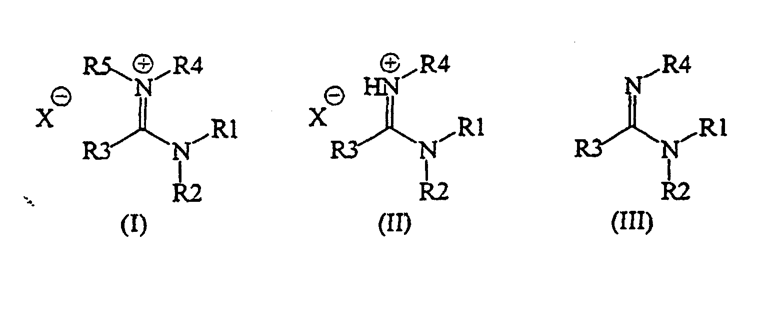 in denen die Reste R¹, R² und R⁴ lineare oder verzweigte Alkylketten mit 1 bis 20 Kohlenstoffatomen oder cycloaliphatische Reste mit einer Ringgröße von 5 bis 8 Kohlenstoffatomen, wobei die Ringe durch Heteroatome unterbrochen sein können, oder R¹ und R² unsubstituierte oder substituierte aromatische Reste sind oder gemeinsam eine Kette aus 4 oder 5 Methylengruppen bilden;
R³ ein Wasserstoffatom oder ein verzweigter oder unverzweigter Alkylrest mit 1 bis 6 Kohlenstoffatomen oder ein Cycloalkylrest mit 3 bis 6 Kohlenstoffatomen und
R⁵ in Verbindung I eine verzweigte oder unverzweigte Alkylkette mit 1 bis 6 Kohlenstoffatomen und X⁻ ein Halogenid ist.

2. Verfahren nach Anspruch 1, **dadurch gekennzeichnet, daß** die Reste R¹, R², R⁴ und R⁵ in Verbindung I unabhängig voneinander Methyl- oder Ethylgruppen sind und der Rest R³ Wasserstoff ist.

3. Verfahren nach Anspruch 2, **dadurch gekennzeichnet, daß** eine katalytische Menge einer Verbindung ausgewählt aus N,N,N'-Trimethylformamidin, N,N,N'-Trimethylformamidin-hydrochlorid, N,N,N',N'-Tetramethylformamidiniumhydrochlorid und N,N-Diethyl-N',N'-Dimethyl-formamidiniumhydrochlorid eingesetzt wird.

4. Verfahren nach einem der Ansprüche 1 bis 3, **dadurch gekennzeichnet, daß** die katalytische Menge 0,5 bis 5 mol-%, bezogen auf die eingesetzte Menge an Carbonsäure, an Carbonsäureanhydrid, an cyclischem Carbonsäureester bzw. an Sulfonsäure, beträgt.

5. Verfahren nach einem der Ansprüche 1 bis 4, **dadurch gekennzeichnet, daß** das Verfahren in Abwesenheit eines Lösungsmittels oder mit dem gewünschten Carbonsäurechlorid als Lösungsmittel durchgeführt wird.

6. Verfahren nach einem der Ansprüche 1 bis 5, **dadurch gekennzeichnet, daß** das Verfahren bei einer Temperatur von 40 bis 120°C durchgeführt wird.

7. Verwendung einer Verbindung aus der Gruppe bestehend aus N,N,N',N'tetrasubstituierten Amidiniumhalogeniden (I), N,N,N'-trisubstituierten Amidiniumhydrohalogeniden (II) und N,N,N'-trisubstituierten Amidinen (III) der allgemeinen Formel 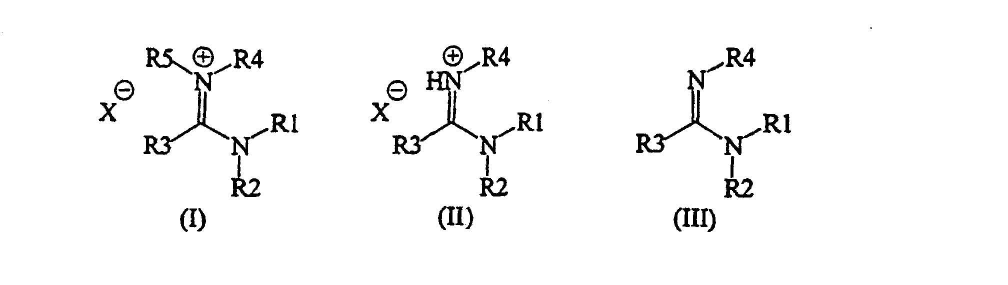 in denen die Reste R¹, R² und R⁴ lineare oder verzweigte Alkylketten mit 1 bis 20 Kohlenstoffatomen oder cycloaliphatische Reste mit einer Ringgröße von 5 bis 8 Kohlenstoffatomen, wobei die Ringe durch Heteroatome unterbrochen sein können, oder R¹ und R² unsubstituierte oder substituierte aromatische Reste sind oder gemeinsam eine Kette aus 4 oder 5 Methylengruppen bilden;
R³ ein Wasserstoffatom oder ein verzweigter oder unverzweigter Alkylrest mit 1 bis 6 Kohlenstoffatomen oder ein Cycloalkylrest mit 3 bis 6 Kohlenstoffatomen und
R⁵ in Verbindung I eine verzweigte oder unverzweigte Alkylkette mit 1 bis 6 Kohlenstoffatomen und X⁻ ein Halogenid ist,
als Katalysator in einem Verfahren zur Herstellung von Säurechloriden durch Umsetzung von Carbonsäuren, Carbonsäureanhydriden, cyclischen Carbonsäureestern (Lactonen) oder Sulfonsäuren mit Phosgen.

## Claims

1. A process for the preparation of acid chlorides by reaction of carboxylic acids, carboxylic anhydrides, cyclic carboxylic esters or sulfonic acids with phosgene in the presence of a catalytic amount of a compound from the group consisting of N,N,N',N'-tetrasubstituted amidinium halides (I), N,N,N'-trisubstituted amidinium hydrohalides (II) and N,N,N'-trisubstituted amidines (III) of the formula 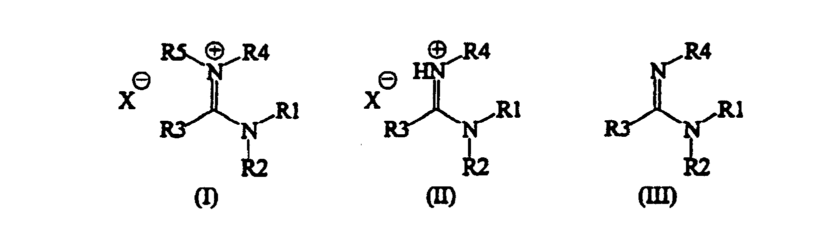 in which the radicals R¹, R² and R⁴ are linear or branched alkyl chains having from 1 to 20 carbon atoms or cycloaliphatic radicals having a ring size of from 5 to 8 carbon atoms, where the rings can be interrupted by heteroatoms, or R¹ and R² are unsubstituted or substituted aromatic radicals or together form a chain of 4 or 5 methylene groups;
R³ is a hydrogen atom or a branched or unbranched alkyl radical having from 1 to 6 carbon atoms or a cycloalkyl radical having from 3 to 6 carbon atoms and
R⁵ in compound I is a branched or unbranched alkyl chain having from 1 to 6 carbon atoms, and X⁻ is a halide.

2. A process as claimed in claim 1, wherein the radicals R¹, R², R⁴ and R⁵ in compound I are independently of one another methyl or ethyl groups, and the radical R³ is hydrogen.

3. A process as claimed in claim 2, wherein a catalytic amount of a compound chosen from N,N,N'-trimethylformamidine, N,N,N'-trimethylformamidine hydrochloride, N,N,N',N'-tetramethylformamidinium hydrochloride and N,N-diethyl-N',N'-dimethylform-amidinium hydrochloride is used.

4. A process as claimed in any of claims 1 to 3, wherein the catalytic amount is from 0.5 to 5 mol%, based on the amount of carboxylic acid, of carboxylic anhydride, of cyclic carboxylic ester or of sulfonic acid used.

5. A process as claimed in any of claims 1 to 4, wherein the process is carried out in the absence of a solvent or with the desired carbonyl chloride as solvent.

6. A process as claimed in any of claims 1 to 5, wherein the process is carried out at a temperature of from 40 to 120°C.

7. The use of a compound from the group consisting of N,N,N',N'-tetrasubstituted amidinium halides (I), N,N,N'-trisubstituted amidinium hydrohalides (II) and N,N,N'-trisubstituted amidines (III) of the formula 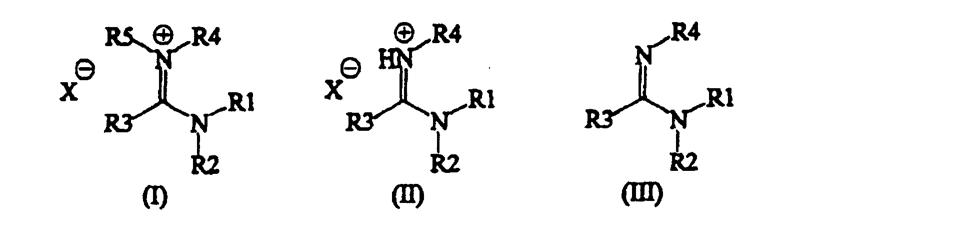 in which the radicals R¹, R² and R⁴ are linear or branched alkyl chains having from 1 to 20 carbon atoms or cycloaliphatic radicals having a ring size of from 5 to 8 carbon atoms, where the rings can be interrupted by heteroatoms, or R¹ and R² are unsubstituted or substituted aromatic radicals or together form a chain of 4 or 5 methylene groups;
R³ is a hydrogen atom or a branched or unbranched alkyl radical having from 1 to 6 carbon atoms or a cycloalkyl radical having from 3 to 6 carbon atoms and
R⁵ in compound I is a branched or unbranched alkyl chain having from 1 to 6 carbon atoms, and X⁻ is a halide,
as catalyst in a process for the preparation of acid chlorides by reaction of carboxylic acids, carboxylic anhydrides, cyclic carboxylic esters (lactones) or sulfonic acids with phosgene.

## Revendications

1. Procédé de préparation de chlorures d'acide par réaction d'acides carboxyliques, d'anhydrides d'acide carboxylique, d'esters cycliques d'acide carboxylique ou d'acides sulfoniques, avec du phosgène en présence d'une quantité catalytique d'un composé du groupe consistant en les halogénures d'amidinium N,N,N',N',-tétrasubstitué (I), les hydrohalogénures d'amidinium N,N,N'-trisubstitué (II) et les amidines N,N,N'-trisubstituées (III), des formules générales suivantes : 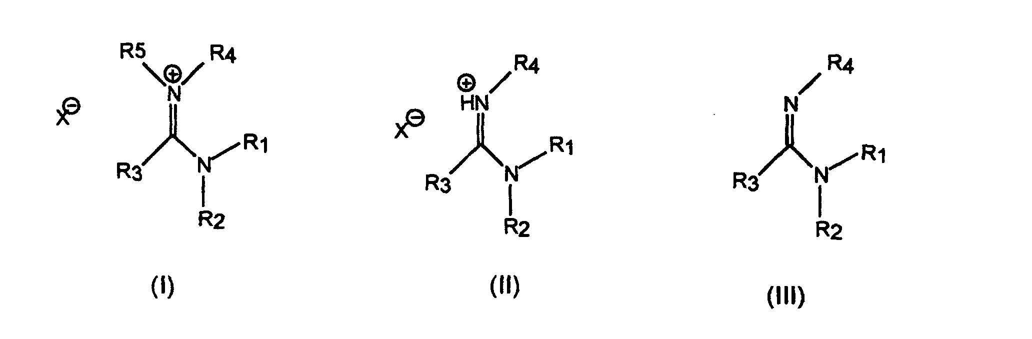 dans lesquelles les restes R₁, R₂ et R₄ représentent des chaînes alkyle linéaires ou ramifiées avec 1 à 20 atomes de carbone ou des restes cycloaliphatiques avec une taille de cycle allant de 5 à 8 atomes de carbone, où le cycle peut être interrompu par des hétéroatomes, ou R₁ et R₂ sont des restes aromatiques non substitués ou substitués ou forment ensemble, une chaîne de 4 ou 5 radicaux méthylène ;
R₃ représente un atome de carbone ou un reste alkyle ramifié ou non ramifié avec 1 à 6 atomes de carbone ou un reste cycloalkyle avec 3 à 6 atomes de carbone, et
R₅ dans le composé I, représente une chaîne alkyle ramifiée ou non ramifiée avec 1 à 6 atomes de carbone, et
X⁻ est un halogénure.

2. Procédé selon la revendication 1, **caractérisé en ce que** les restes R₁, R₂, R₄ et R₅ dans le composé I, sont indépendamment l'un de l'autre, le radical méthyle ou éthyle et le reste R₃ est hydrogène.

3. Procédé selon la revendication 2, **caractérisé** en ce l'on met en oeuvre une quantité catalytique d'un composé choisi parmi la N,N,N'-triméthylformamidine, le chlorhydrate de N,N,N'-triméthylformamidine, le chlorhydrate de N,N,N',N'-tétraméthylformamidinium et le chlorhydrate de N,N-diéthyl-N',N'-diméthylformamidinium.

4. Procédé selon l'une quelconque des revendications 1 à 3, **caractérisé en ce que** la quantité catalytique se situe dans l'intervalle allant de 0,5 à 5% en moles, sur base de la quantité mise en oeuvre d'acide carboxylique, d'anhydride d'acide carboxylique, d'ester cyclique d'acide carboxylique ou d'acide sulfonique.

5. Procédé selon l'une quelconque des revendications 1 à 4, **caractérisé en ce que** le procédé est réalisé en l'absence de solvant ou avec le chlorure d'acide carboxylique souhaité comme solvant.

6. Procédé selon l'une quelconque des revendications 1 à 5, **caractérisé en ce que** le procédé est réalisé à une température allant de 40 à 120°C.

7. Utilisation d'un composé du groupe consistant en les halogénures d'amidinium N,N,N',N',-tétrasubstitué (I), les hydrohalogénures d'amidinium N,N,N'-trisubstitué (II) et les amidines N,N,N'-trisubstituées (III), des formules générales suivantes : 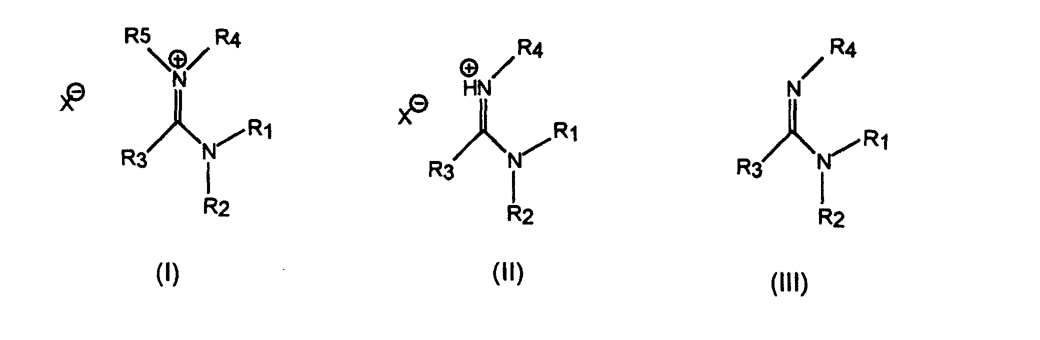 dans lesquelles les restes R₁, R₂ et R₄ représentent des chaînes alkyle linéaires ou ramifiées avec 1 à 20 atomes de carbone ou des restes cycloaliphatiques avec une taille de cycle allant de 5 à 8 atomes de carbone, où le cycle peut être interrompu par des hétéroatomes, ou R₁ et R₂ sont des restes aromatiques non substitués ou substitués ou forment ensemble, une chaîne de 4 ou 5 radicaux méthylène ;
R₃ représente un atome de carbone ou un reste alkyle ramifié ou non ramifié avec 1 à 6 atomes de carbone ou un reste cycloalkyle avec 3 à 6 atomes de carbone, et
R₅ dans le composé I, représente une chaîne alkyle ramifiée ou non ramifiée avec 1 à 6 atomes de carbone, et
X⁻ est un halogénure,
comme catalyseur dans un procédé pour la préparation de chlorures d'acide par réaction d'acides carboxyliques, d'anhydrides d'acide carboxylique, d'esters cycliques d'acide carboxylique (lactones) ou d'acides sulfoniques, avec du phosgène.
